**Europäisches Patentamt**

**European Patent Office**

**Office européen des brevets**

(19)

(11) Numéro de publication : **0 295 991 B1**

(12)

# FASCICULE DE BREVET EUROPEEN

(45) Date de publication du fascicule du brevet :
**28.08.91 Bulletin 91/35**

(51) Int. Cl.⁵ : **G01T 1/164, G01T 1/17**

(21) Numéro de dépôt : **88401290.7**

(22) Date de dépôt : **26.05.88**

(54) Procédé de localisation d'un rayonnement nucléaire.

(30) Priorité : **27.05.87 FR 8707481**

(43) Date de publication de la demande :
**21.12.88 Bulletin 88/51**

(45) Mention de la délivrance du brevet :
**28.08.91 Bulletin 91/35**

(84) Etats contractants désignés :
**DE GB NL**

(56) Documents cités :
**FR-A- 2 387 559**
**US-A- 3 745 345**
**US-A- 4 223 388**

(73) Titulaire : **COMMISSARIAT A L'ENERGIE ATOMIQUE Etablissement de Caractère Scientifique Technique et industriel 31/33, rue de la Fédération F-75015 Paris (FR)**

(72) Inventeur : **Tararine, Michel 6, rue du Four F-92330 Sceaux (FR)**
Inventeur : **Thévenin, Bernard 1 Chemin la Bourrelière F-38120 Saint Egrève (FR)**
Inventeur : **Marcaud, Monique 1 Chemin de la Croix Gorge F-38120 Saint Egrève (FR)**

(74) Mandataire : **Mongrédien, André et al c/o BREVATOME 25, rue de Ponthieu F-75008 Paris (FR)**

## Description

La présente invention a pour objet un procédé de localisation d'un rayonnement nucléaire.

Elle trouve son application notamment dans le domaine médical où des dispositifs de formation d'image de rayonnement sont utilisés comme aide au diagnostic. De tels dispositifs sont appelés généralement caméra à scintillation, caméra à rayons gamma, ou caméra de type ANGER. Les documents US-A-3011057, US-A-3745345 et FR-A-2412856 décrivent de telles caméras.

L'application de l'invention dans le domaine médical implique l'administration par injection dans une veine d'un patient d'une petite dose d'un radio-isotope (une substance radioactive qui émet des rayons gamma). La circulation sanguine distribue la dose dans le corps et un transducteur d'une sensibilité appropriée enregistre le développement de cette distribution.

Les régions du corps qui ont une grande affinité pour l'isotope ou une irrigation sanguine riche apparaissent comme des sources brillantes ou fortement éclairées tandis qu'inversement, les régions de faible affinité ou de faible irrigation sanguine apparaissent sombres. De cette manière, toute partie du corps ou un organe spécifique peut être soumis à une investigation clinique d'une manière sûre, fiable et sans intervention.

Une caméra à rayons gamma comprend une tête de détection composée généralement d'un collimateur pour focaliser les rayons gamma émis par le patient, d'un cristal pour transformer les photons gamma en photons lumineux, ou scintillations, et d'un réseau de tubes photomultiplicateurs pour transformer chaque scintillation en une impulsion électrique analogique, appelée aussi contribution électrique.

Elle comprend en outre un codeur de position pour produire des signaux de coordonnées X et Y du lieu où s'est produite une scintillation, à partir des impulsions électriques analogiques fournies par les tubes photomultiplicateurs.

Une caméra à rayons gamma comprend enfin généralement un oscilloscope cathodique commandé par les signaux de coordonnées X et Y, et par un signal de validation Z produit par le codeur de position à partir des impulsions fournies par les tubes photomultiplicateurs, lorsque l'énergie de la scintillation appartient à une bande d'énergie prédéterminée. Un rayonnement gamma atteignant le cristal est ainsi visualisé par un point lumineux sur l'écran de l'oscilloscope cathodique.

Pour produire une image de rayonnement représentatrice de tous les rayonnements nucléaires reçus, une caméra à rayons gamma peut également comporter un dispositif photographique pour former une image de l'organe observé par accumulation d'un grand nombre de points lumineux produits sur l'écran de l'oscilloscope cathodique, et un moyen de traitement numérique des images, notamment en vue d'obtenir des tomographies de l'organe observé.

Lorsqu'un rayonnement gamma atteint le cristal, une scintillation est produite. On sait qu'une telle scintillation est vue simultanément par plusieurs tubes photomultiplicateurs, de l'ordre de 6 à 10 tubes. La détermination de l'emplacement de la scintillation est obtenue en calculant l'emplacement du barycentre des contributions électriques délivrées par l'ensemble des tubes photomultiplicateurs excités par cette scintillation.

Ce calcul est effectué classiquement, comme décrit dans le document US-A-3011057, en utilisant plusieurs jeux de résistances électriques dont les valeurs sont fonction des positions des tubes photomultiplicateurs auxquelles elles sont raccordées. Ces positions sont définies par rapport à un repère de référence d'axes Ox et Oy. Dans chaque jeu de résistances, il y a une résistance par tube photomultiplicateur, chaque résistance étant reliée par une extrémité à un tube photomultiplicateur et par une autre extrémité à un point commun. Le signal délivré sur ce point commun est ainsi une pondération des signaux délivrés par les tubes photomultiplicateurs.

Le codeur de position comprend généralement 4 jeux différents de résistances qui délivrent des signaux analogiques notés $X^+$, $X^-$, $Y^+$ et $Y^-$. Il peut éventuellement comprendre un cinquième jeu de résistances pour délivrer le signal de validation Z, représentatif de l'énergie de la scintillation.

Selon l'art antérieur, des signaux analogiques de coordonnées X, Y sont déduits, par un calcul analogique, des signaux $X^+$, $X^-$, $Y^+$, $Y^-$ et éventuellement du signal Z.

Les relations généralement utilisées pour le calcul de la coordonnée X sont l'une des suivantes :

$$X = \frac{X^+ - X^-}{X^+ + X^-} \quad \text{ou} \quad X = \frac{X^+ - X^-}{X^+ + X^- + Y^+ + Y^-} \quad \text{ou} \quad X = \frac{X^+ - X^-}{Z}$$

Des relations symétriques sont utilisées pour le calcul de la coordonnée Y.

Les signaux de coordonnées X, Y obtenus, et le signal Z, le cas échéant, sont ensuite appliqués sur les entrées de convertisseurs analogiques-numériques pour être numérisés.

Cette technique présente l'inconvénient de nécessiter des convertisseurs analogiques-numériques ayant une linéarité différentielle très importante.

Dans la demande de brevet français n° 8707482 intitulée "Dispositif de localisation de rayonnement nucléaire, et dispositif de formation d'image de rayonnement incluant un tel dispositif de localisation" déposée au nom du demandeur à la même date que la présente demande de brevet, il est proposé de remédier à cette difficulté en numérisant les signaux $X^+$, $X^-$, $Y^+$, $Y^-$ puis en calculant numériquement les coordonnées X, Y par les relations indiquées au-dessus.

Les relations définissant les coordonnées X, Y comportent une opération de division. En pratique, ce calcul est ramené à une opération de multiplication entre deux opérandes, le premier opérande $O_1$ étant $X^+ - X^-$ (pour le calcul de la coordonnée X) ou $Y^+ - Y^-$ (pour le calcul de la coordonnée Y), et le second opérande $O_2$ étant inversement proportionnel à l'énergie D du rayonnement nucléaire détecté. Cette énergie D peut être prise égale à l'une des valeurs suivantes : $X^+ + X^-$, $X^+ + X^- + Y^+ + Y^-$ ou Z. L'opérande $O_2$ est de la forme K/D, où K est une constante qui permet d'avoir $O_2$ sous forme entière, et peut être stockée dans une table dont la cellule d'adresse D contient K/D.

Le nombre de bits $N_1$ de l'opérande $O_1$ est fonction de la précision qu'il est possible d'avoir sur les données numériques $X^+$, $X^-$, $Y^+$, $Y^-$. Celle-ci est limitée par le fait que les valeurs des résistances électriques utilisées pour produire les signaux analogiques $X^+$, $X^-$, $Y^+$, $Y^-$ présentent toujours une certaine dispersion par rapport à leur valeur nominale. Pour cette raison, le nombre maximum de bits utilisé pour coder l'opérande $O_1$ est de l'ordre de 12 à 16 bits.

De préférence, l'opérande $O_2$ est codé sur un nombre de bits $n_2$ proche de $n_1$. La donnée D étant codée sur $n_1$ bits (ou éventuellement $n_1 + 1$ bits), la constante K doit donc être codée sur environ $n_1 + n_2$ bits.

Les coordonnées X, Y sont obtenues en multipliant les opérandes $O_1$ et $O_2$ entre eux. Le résultat est codé sur $n_1 + n_2$ bits, soit au moins 24 bits. En fait, pour un cristal scintillateur classique constitué d'un disque d'environ 400 mm de diamètre, tous ces bits ne sont pas significatifs. Si on ne garde par exemple que les 12 bits de poids fort, on obtient déjà une précision sur les coordonnées X, Y de 0,1 mm ($400/2^{12}$).

Donc, en pratique, les coordonnées X, Y sont codées sur m bits où $m < n_1 + n_2$, par troncature des bits de rangs inférieurs à $n_1 + n_2 - (m - 1)$ du produit $O_1 \times O_2$.

Le demandeur a mis en oeuvre le procédé de détermination des coordonnées X, Y décrit ci-dessus dans un système de formation d'image de rayonnement. Il a constaté que lorsqu'un rayonnement nucléaire venait exciter uniformément la surface du cristal scintillateur, le système ne produisait pas une image de rayonnement uniforme mais au contraire une image présentant un phénomène de moiré important. Ceci constitue un inconvénient majeur qui peut empêcher une interprétation correcte de l'image de rayonnement produite par un organe analysé.

Le procédé de détermination des coordonnées X, Y décrit ci-dessus fait donc apparaître un nouveau problème technique.

L'apparition de ce phénomène de moiré est lié à l'emploi de données numériques et peut être expliqué de la manière suivante.

Pour une application déterminée, on utilise un rayonnement nucléaire d'énergie fixe ; par exemple, les photons gamma produits par l'isotope radio-actif $^{99}$Te du Technetium ont une énergie de 140 keV. L'opérande $O_2$, qui est inversement proportionnel à l'énergie a donc une valeur fixe.

On sait que les signaux $X^+$, $X^-$ (respectivement $Y^+$, $Y^-$) sont des fonctions linéaires de la coordonnée X (respectivement Y). Donc, l'opérande $O_1$ égal à $X^+ - X^-$ ou $Y^+ - Y^-$ varie par pas constant P1 et le produit $O_1 \times O_2$ varie par pas constant égal à $P1.O_2$. Par ailleurs, pour mesurer les coordonnées X, Y, on tronque le produit $O_1 \times O_2$ en supprimant les bits de rangs inférieurs à $n_1 + n_2 - (m - 1)$. Ceci revient à mesurer les coordonnées X, Y par pas P2 égal à

$$2^{n_1 + n_2 - m}.$$

Les pas $P1.O_2$ et P2 créent chacun une périodicité dans la gamme des valeurs de la coordonnée X. Il en est de même pour la coordonnée Y. Si ces pas sont différents, il y a apparition d'un battement, donc d'une périodicité, ce qui se traduit visuellement par un phénomène de moiré.

En pratique, le phénomène est quelque peu atténué car l'énergie du rayonnement nucléaire n'est pas fixe, mais présente au contraire une certaine fluctuation statistique. L'opérande $O_2$ n'est donc pas constant, mais varie autour d'une valeur moyenne. Il en résulte que le pas $P1.O_2$ n'est pas non plus constant.

Les mots de poids faible des coordonnées X, Y c'est-à-dire les $n_1 + n_2 - m$ bits de poids faible qui disparaissent par troncature, sont alors distribués aléatoirement sur un intervalle qui est fonction de la largeur de la fenêtre en énergie du rayonnement nucléaire. Lorsque cet intervalle est suffisamment élevé, les mots de poids faible des coordonnées X, Y sont distribués aléatoirement sur toutes ses valeurs possibles ; les battements sont alors distribués sur un intervalle plus grand que la plus grande valeur des coordonnées X, Y et les battements ne sont plus visibles.

Le phénomène de moiré s'atténue également lorsque l'énergie du rayonnement nucléaire est plus importante. En effet, le pas P1 de variation des différences $X^+ - X^-$ et $Y^+ - Y^-$ est plus grand, ce qui diminue la fréquence spatiale des battements.

En pratique, la fluctuation statistique de l'énergie du rayonnement nucléaire et l'énergie du rayonnement nucléaire elle-même ne sont pas suffisantes pour masquer totalement le phénomène de moiré.

L'invention a précisément pour but de supprimer le phénomène de moiré, dont l'apparition est liée au calcul numérique des coordonnées X, Y à partir des données numériques $X^+$, $X^-$, $Y^+$ et $Y^-$.

L'invention consiste à supprimer les battements en introduisant un élément aléatoire dans le calcul des coordonnées X, Y.

Selon un premier mode de réalisation, l'opérande $O_1$ est constitué par la donnée numérique $X^+ - X^-$ (ou $Y^+ - Y^-$) complétée à droite par p bits aléatoires ($p \geq 1$).

Selon un second mode de réalisation, on ajoute 1 de manière aléatoire à la coordonnée X, Y obtenue après troncature.

De manière précise, l'invention a pour objet un procédé de localisation d'un rayonnement nucléaire détecté par une tête de détection comprenant un cristal pour produire des photons lumineux en réponse à un rayonnement nucléaire reçu, et un ensemble de transducteurs pour délivrer chacun un signal électrique en réponse aux photons lumineux émis, lesdits signaux électriques étant combinés pour produire deux couples de données numériques $X^+$, $X^-$ et $Y^+$, $Y^-$ qui sont fonction respectivement des coordonnées X, Y du lieu du cristal excité par ledit rayonnement nucléaire, ledit procédé consistant à calculer numériquement lesdites coordonnées X, Y exprimées sur m bits, en fonction desdites données numériques exprimées sur n bits,

— à déterminer une donnée numérique A, exprimée sur N bits, inversement proportionnelle à l'énergie du rayonnement nucléaire reçu,

— à déterminer une donnée numérique B par différence des données d'un couple de données numériques,

— à produire une donnée numérique C en complétant B à droite par p bits ($p \geq 1$) de valeurs aléatoires,

— à multiplier les données numériques A et C pour produire une donnée numérique exprimée sur N + n + p bits, et à conserver les m bits de poids forts de ladite donnée numérique, ces m bits étant la valeur de la coordonnée X lorsque B est égal à $X^+ - X^-$ et Y lorsque B est égal à $Y^+ - Y^-$.

Selon le second mode de réalisation,

— on détermine une donnée numérique A, exprimée sur N bits, inversement proportionnelle à l'énergie du rayonnement nucléaire reçu,

— on détermine une donnée numérique B par différence des données d'un couple de données numériques,

— on multiple les données numériques A et B pour produire une donnée numérique exprimée sur N + n bits,

— on produit une donnée numérique F égale aux m bits de poids forts de ladite donnée numérique, et on ajoute 1 de manière aléatoire à ladite donnée F, le résultat étant la valeur de la coordonnée X lorsque B est égal à $X^+ - X^-$ et Y lorsque B est égal à $Y^+ - Y^-$.

Les caractéristiques et avantages de l'invention ressortiront mieux de la description qui va suivre, donnée à titre illustratif mais non limitatif, en référence aux dessins annexés, sur lesquels :

— la figure 1 montre la variation des données numériques $X^+$ et $X^-$ avec la valeur de la coordonnée X et de l'énergie E,

— la figure 2 illustre le procédé de l'invention selon un premier mode de réalisation, et

— la figure 3 illustre le procédé de l'invention selon un second mode de réalisation.

L'invention se rapporte à la localisation d'un rayonnement nucléaire et s'applique notamment aux dispositifs de formation d'image de rayonnement décrits dans les documents US-A-3011057, US-A-3745345 et FR-A-241856 déjà cités.

Plus précisément, l'invention concerne le calcul numérique des coordonnées X, Y du lieu d'excitation d'un cristal, faisant partie d'une tête de détection d'un dispositif de formation d'image de rayonnement, à partir de données $X^+$, $X^-$, $Y^+$, $Y^-$ sous forme numérique.

Le procédé de l'invention peut avantageusement être mis en oeuvre par le moyen de calcul numérique du codeur de position décrit dans la demande de brevet français intitulée "dispositif de localisation de rayonnement nucléaire, et dispositif de formation d'image de rayonnement" également déjà citée.

La détermination des coordonnées X, Y est faite classiquement à partir de signaux $X^+$, $X^-$, $Y^+$, $Y^-$ qui représentent des sommes pondérées de signaux délivrés par des transducteurs de type tube photomultiplicateur inclus dans un dispositif de formation d'image de rayonnement.

Dans la demande de brevet français citée ci-dessus, le demandeur a proposé de coder ces signaux sous forme de données numériques.

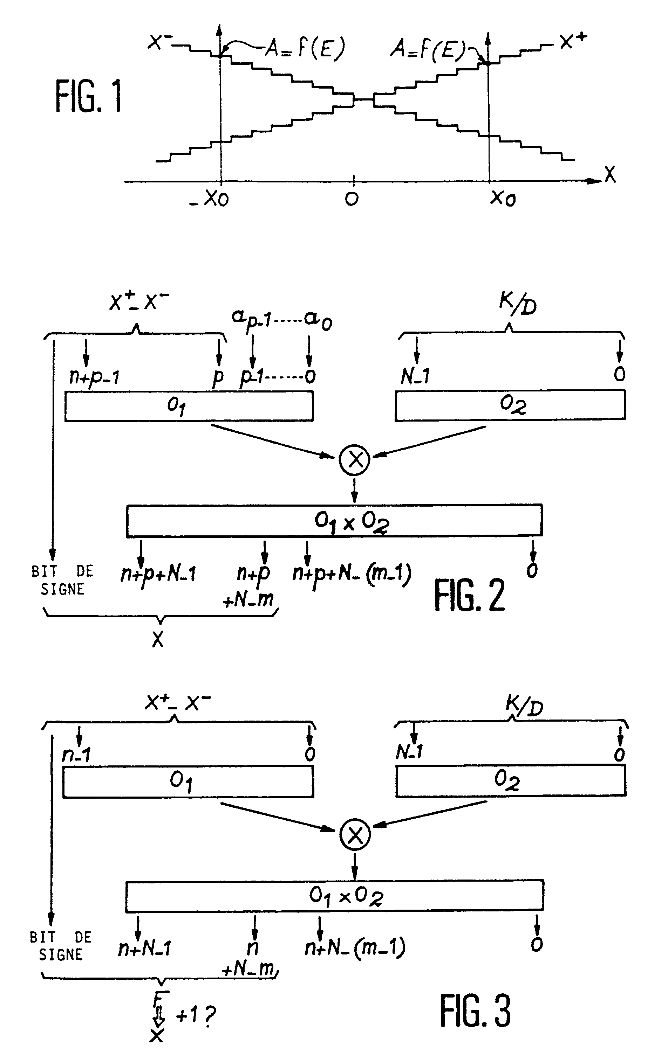

La variation des données X+, X− est représentée sur la figure 1. Ces données varient par pas constant en fonction de la coordonnée X et de l'énergie E. Les données X+, X− sont positives sur l'intervalle [ − X₀, X₀] qui représente la gamme d'amplitude de X (le point 0 étant au milieu de l'image).

La figure 1 montre que la différence X+ − X− peut être codée sur le même nombre de bits n que les données X+ et X−, avec l'ajout d'un bit de signe. On constate également que X+ + X− est constant mais ceci n'est en réalité qu'une approximation car la pente des signaux X+, X− varie avec l'énergie du rayonnement nucléaire.

La figure 2 illustre schématiquement un premier mode de réalisation du procédé de l'invention.

La coordonnée X est calculée comme fonction du produit d'une donnée numérique A, égale à la différence X+ − X−, et d'une donnée numérique B, inversement proportionnelle à l'énergie du rayonnement nucléaire reçu. La donnée numérique B est de la forme K/D, où D peut être égal de manière connue à X+ + X−, X+ + X− + Y+ + Y− ou Z, et où K est une constante choisie pour que le rapport K/D soit un entier.

Selon l'invention, on produit un premier opérande $O_1$ en complétant la donnée X+ − X− par p bits $a_{p−1}$,..., $a_0$ dont les valeurs sont déterminées de manière aléatoire. Le nombre de bits aléatoires p est de l'ordre de quelques unités au plus. On produit également un second opérande $O_2$ en calculant D suivant l'une des relations ci-dessus puis en déterminant K/D, par exemple en accédant à une table stockée dans une mémoire comprenant un ensemble de cellules de mémorisation et dont la cellule de mémorisation d'adresse D contient K/D.

Les deux opérandes $O_1$ et $O_2$ sont par exemple exprimés sur 16 bits avec n = 12, p = 4 et N = 16.

Les produits sont réalisés à titre d'exemple indicatif au moyen d'un multiplieur 2 × 16bits. On pourra par exemple utiliser le multiplieur AMD 29516 ou tout autre multiplieur commercialisé.

La coordonnée X est définie par le produit $O_1 × O_2$. Le nombre m de bits de poids forts conservés fixe la résolution sur l'axe de coordonnée. Pour un cristal scintillateur dont le rayon est 20 cm, la résolution en X est de 0,1 mm avec m = 11.

Le signe de la coordonnée X est déterminé par un bit de signe supplémentaire qui est le bit de signe de la différence X+ − X−.

L'obtention de p bits aléatoires est réalisée de manière classique, soit à titre d'exemple, en utilisant un compteur actionné en permanence par une horloge et en effectuant des tirages à chaque événement de sortie du compteur, le temps d'arrivée des événements étant aléatoire.

Ce premier mode de réalisation de l'invention présente l'avantage de pouvoir être mis en oeuvre très facilement.

La figure 3 illustre un autre mode de réalisation du procédé de l'invention. La coordonnée X est calculée en fonction du produit des mêmes données numériques A et B que dans le mode de réalisation précédent.

Une première différence réside en ce qu'on ne complète pas la donnée A, mais que les opérandes $O_1$ et $O_2$ sont respectivement égaux à X+ − X− et K/D.

La coordonnée X est définie par le produit $O_1 × O_2$. Comme dans le mode de réalisation précédent, on ne conserve qu'un nombre suffisant m de bits de poids forts pour atteindre la résolution désirée. A cette donnée numérique F, exprimée sur m bits, on ajoute aléatoirement la valeur 1. Le résultat constitue la coordonnée X.

L'ajout aléatoire de la valeur 1 peut être réalisé par exemple avec une probabilité fonction de, par exemple proportionnelle à, la valeur des bits de rangs inférieur ou égal à n + N − (m − 1) du produit $O_1 × O_2$.

Ce second mode de réalisation permet d'annuler les battements dont la périodicité est plus faible que le pas P2 de mesure de la coordonnée X, c'est-à-dire plus faible que $2^{n+N−m}$. En revanche, les battements de périodicité supérieure ne disparaissent pas.

Les figures 1 à 3 illustrent la détermination de la coordonnée X. La coordonnée Y s'obtient de manière analogue.

Les variations des données Y+, Y− en fonction de la coordonnée Y sont semblables aux variations des données X+, X− en fonction de la coordonnée X représentées sur la figure 1.

La coordonnée Y est obtenue comme fonction du produit de la donnée A égale à Y+ − Y− (au lieu de X+ − X− pour la coordonnée X) et de la donnée B égale à K/D, où D est généralement choisi égal à l'une des valeurs suivantes : Y+ − Y−, X+ + X− + Y+ + Y− ou Z.

## Revendications

1. Procédé de localisation d'un rayonnement nucléaire détecté par une tête de détection comprenant un cristal pour produire des photons lumineux en réponse à un rayonnement nucléaire reçu, et un ensemble de transducteurs pour délivrer chacun un signal électrique en réponse aux photons lumineux émis, lesdits signaux électriques étant combinés pour produire deux couples de données numériques X+, X− et Y+, Y− qui sont fonction respectivement des coordonnées X, Y du lieu du cristal excité par ledit rayonnement nucléaire et de l'énergie dudit rayonnement nucléaire, ledit procédé consistant à calculer numériquement lesdites coordonnées X, Y, exprimées sur m bits, en fonction desdites données numériques exprimées sur n bits,

— à déterminer une donnée numérique A, exprimée sur N bits, inversement proportionnelle à l'énergie du rayonnement nucléaire reçu,

— à déterminer une donnée numérique B par différence des données d'un couple de données numériques,

— à produire une donnée numérique C en complétant la donnée numérique B à droite par p bits $a_{p-1}$,..., $a_0$ ; $p \geq 1$, de valeurs aléatoires,

— à multiplier les données numériques A et C pour produire une donnée numérique exprimée, sur N + n + p bits, et à conserver les m bits de poids forts de ladite donnée numérique, lesdits m bits représentant la valeur de la coordonnée X lorsque B est égal à $X^+ - X^-$ et Y lorsque B est égal à $Y^+ - Y^-$.

2. Procédé de localisation d'un rayonnement nucléaire détecté par une tête de détection comprenant un cristal pour produire des photons lumineux en réponse à un rayonnement nucléaire reçu, et un ensemble de transducteurs pour délivrer chacun un signal électrique en réponse aux photons lumineux émis, lesdits signaux électriques étant combinés pour produire deux couples de données numériques $X^+$, $X^-$ et $Y^+$, $Y^-$ qui sont fonction respectivement des coordonnées X, Y du lieu du cristal excité par ledit rayonnement nucléaire et de l'énergie rayonnement nucléaire, ledit procédé consistant à calculer numériquement lesdites coordonnées X, Y, exprimées sur m bits, en fonction desdites données numériques exprimées sur n bits.

— à déterminer une donnée numérique A, exprimée sur N bits, inversement proportionnelle à l'énergie du rayonnement nucléaire reçu,

— à déterminer une donnée numérique B par différence des données d'un couple de données numériques,

— à multiplier les données numériques A et B pour produire une donnée numérique exprimée sur N + n bits,

— à produire une donnée numérique F égale aux m bits de poids forts de ladite donnée numérique, et on ajoute la valeur 1 de manière aléatoire à ladite donnée numérique F, le résultat représentant la valeur de la coordonnée X lorsque B est égal à $X^+ - X^-$ et Y lorsque B est égal à $Y^+ - Y^-$.

3. Procéddé selon la revendication 2, caractérisé en ce qu'on ajoute la valeur 1 à la donnée numérique F avec une probabilité fonction de la valeur des bits de rang inférieur ou égal à N + n – m du produit A.B exprimé sur N + n bits.

4. Procédé selon l'une quelconque des revendications 1 à 3, caractérisé en ce que la donnée numérique A est de la forme K/D, où K est une constante et D est une donnée numérique proportionnelle à l'énergie du rayonnement nucléaire reçu, ladite donnée numérique A étant déterminée par lecture dans une table adressée par la donnée numérique D et contenant à l'adresse D la valeur K/D.

5. Procédé selon la revendication 4, caractérisé en ce que pour le calcul de la coordonnée X (respectivement Y), on choisit D égal à $X^+ + X^-$ (respectivement $Y^+ + Y^-$).

6. Procédé selon la revendication 4, caractérisé en ce que pour le calcul des coordonnées X, Y, on choisit la même valeur D égale à $X^+ + X^- + Y^+ + Y^-$.

7. Procédé selon la revendication 4, caractérisé en ce que les signaux électriques délivrés par les transducteurs sont en outre combinés pour produire une donnée numérique Z fonction de l'énergie du rayonnement nucléaire reçu, et on choisit D égal à ladite donnée numérique Z.

## Patentansprüche

1. Verfahren zum Lokalisieren einer Kernstrahlung, die durch einen Erfassungskopf erfaßt wird mit einem Kristall, um leuchtende Photonen in Antwort auf eine auftreffende Kernstrahlung zu erzeugen, und einer Übertrageranordnung, um jeweils ein elektrisches Signal in Antwort auf die ausgesendeten leuchtenden Photonen zu liefern, wobei die elektrischen Signale kombiniert werden, um Paare von numerischen Werten $X^+$, $X^-$ und $Y^+$, $Y^-$ zu erzeugen, die Funktionen der Koordinaten X, Y des Ortes des kristalls sind, der von der Kernstrahlung angeregt wird bzw. der Energie der Kernstrahlung, wobei das Verfahren daraus besteht, numerisch die genannten Koordinaten X, Y, ausgedrückt auf m Bits zu berechnen, in Abhängigkeit der numerischen Werte, ausgedrückt in n Bits,

Bestimmen eines numerischen Wertes A, ausgedrückt in N Bits, der umgekehrt proportional zur empfangenen Kernstrahlungsenergie ist,

Bestimmen eines numerischen Wertes B durch Abziehen der Werte von einem numerischen Wertepaar,

Erzeugen eines numerischen Wertes C unter Vervollständigung des numerischen Wertes B nach rechts durch p Bits $a_{p-1}$,..., $a_0$ ; $p > 1$, von stochastischen Werten,

Multiplizieren der numerischen Werte A und C, um einen numerischen Wert, ausgedrückt in N + n + b Bits zu erzeugen, und Konservieren bzw. Speichern der m Bits von groben Gewichten des genannten nume-

EP 0 295 991 B1

rischen Werts, wobei die m Bits den Wert der Koordinate X darstellen, wenn B gleich X⁺ − X⁻ und Y darstellen, wenn B gleich Y⁺ − Y⁻ ist.

2. Verfahren zum Lokalisieren einer Kernstrahlung, die von einem Erfassungskopf erfaßt wird, der einen Kristall aufweist, um leuchtende Photonen zu erzeugen in Antwort auf eine empfangene Kernstrahlung, und eine Übertrageranordnung aufweist, um jeweils ein elektrisches Signal in Antwort auf die emittierten leuchtenden Photonen zu liefern, wobei die elektrischen Signale kombiniert werden, um zwei Paare von numerischen Werten X⁺, X⁻ und Y⁺, Y⁻ zu erzeugen, die in Abhängigkeit von den Koordinaten X, Y des Kristallortes, der von der Kernstrahlung angeregt wird bzw. der Kernstrahlungsenergie sinnd, wobei das Verfahren besteht aus numerischem Berechnen der Koordinaten X, Y, ausgedrückt in m Bits, in Abhängigkeit der numerischen Werte, ausgedrückt in n Bits,

Bestimmenn eines numerischen Wertes A, ausgedrückt in N Bits, der umgekehrt proportional zur empfangenen Kernstrahlungsenergie ist,

Bestimmen eines numerischen Wertes B durch Abziehen der Werte eines Paares von numerischen Werten,

Multiplizieren der numerischen Werte A und B, um einen numerischen Wert, ausgedrückt in N + n Bits zu erzeugen,

Erzeugen eines numerischen Wertes F gleich m Bits von großen Gewichten des numerischen Wertes und Hinzufügen des Wertes 1 auf stochastische Weise zu dem numerischen Wert F, wobei das Resultat den Wert der Koordinate X darstellt, wenn B gleich X⁺ − X⁻, und Y darstellt, wenn B gleich Y⁺ − Y⁻ ist.

3. Verfahren nach Anspruch 2, **dadurch gekennzeichnet,** daß der Wert 1 zum numerischen Wert F mit einer Wahrscheinlichkeitsfunktion der Werte der Bits von geringerem oder gleichem Rang zu N + n − m des Produktes A.B, ausgedrückt in N + n Bits hinzugefügt wird.

4. Verfahren nach einem der Ansprüche 1 bis 3, **dadurch gekennzeichnet,** daß der numerische Wert A von der Form K/D ist, wobei K eine Konstante ist und D ein numerischer Wert ist, proportional zur empfangenen Kernstrahlungsenergie, wobei der numerische Wert A durch Lesen in einer Tabelle bestimmt wird, adressiert durch den numerischen Wert D und enthalten bei der Adresse D den Wert K/D.

5. Verfahren nach Anspruch 4, **dadurch gekennzeichnet,** daß für die Berechnung der Koordinate X (bzw. Y) D gleich X⁺ + X⁻ (bzw. Y⁺ + Y⁻) gewählt wird.

6. Verfahren nach Anspruch 4, **dadurch gekennzeichnet,** daß für die Berechnung der Koordinaten X, Y derselbe Wert D gleich X⁺ + X⁻ + X⁺ − Y⁻ gewählt wird.

7. Verfahren nach Anspruch 4, **dadurch gekennzeichnet,** daß die von den Übertragern gelieferten elektrischen Signale weiterhin kombiniert werden, um einen numerischen Wert Z als Funktion der empfangenen Kernstrahlungsenergie zu erzeugen, und daß D gleich dem numerischen Wert Z gewählt wird.

## Claims

1. Process for locating a nuclear radiation detected by a detection head incorporating a crystal for producing light photons in response to a received nuclear radiation and a group of transducers, each of which supplies an electric signal in response to the emitted light photons, said electric signals being combined to produce two pairs of digital data X⁺, X⁻ and Y⁺, Y⁻, which are respectively a function of the coordinates X, Y of the location of the crystal excited by said nuclear radiation and the energy of said nuclear radiation, said process consisting of digitally calculating the said coordinates X, Y expressed on m bits, as a function of said digital data expressed on n bits,

— determining a digital data item A, expressed on N bits and inversely proportional to the energy of the received nuclear radiation,

— determining a digital data item B by differentiation of the data of a pair of digital data items,

— producing a digital data item C by completing the digital data item B to the right by p bits $a_{p-1},..., a_0$ (p ≥ 1) with random values and

— multiplying the digital data items A and C to produce a digital data item expressed on N + n + p bits and retaining the m high-order bits of said digital data item, said m bits representing the value of the coordinate X when B is equal to X⁺ − X⁻ and Y when B is equal to Y⁺ − Y⁻.

2. Process for locating a nuclear radiation detected by a detection head incorporating a crystal for producing light photons in response to a received nuclear radiation and a group of transducers, each of which supplies an electric signal in response to the emitted light photons, said electric signals being combined to produce two pairs of digital data X⁺, X⁻ and Y⁺, Y⁻, which are respectively a function of the coordinates X, Y of the location of the crystal excited by said nuclear radiation and the energy of said nuclear radiation, said process consisting of digitally calculating the said coordinates X, Y expressed on m bits, as a function of said digital data expressed

7

on n bits,

— determining a digital data item A, expressed on N bits and inversely proportional to the energy of the received nuclear radiation,

— determining a digital data item B by differentiation of the data of a pair of digital data items,

— multiplying the digital data items A and B to produce a digital data item expressed on N + n bits and

— producing a digital data item F equal to m high-order bits of said digital data item and adding the value 1 in random manner to said digital data item F, the result representing the value of the coordinate X when B is equal to $X^+ - X^-$ and Y when B is equal to $Y^+ - Y^-$.

3. Process according to claim 2, characterized in that the value 1 is added to the digital data item F with a probability which is a function of the bits with an order equal to or below N + n − m of the product A·B expressed on N + n bits.

4. Process according to any one of the claims 1 to 3, characterized in that the digital data item A is of form K/D, in which K is a constant and D a digital data item proportional to the energy of the nuclear radiation received, said digital data item A being determined by reading in a table addressed by the digital data item D and containing the value K/D at address D.

5. Process according to claim 4, characterized in that D is chosen as equal to $X^+ - X^-$ (respectively $Y^+ + Y^-$) for calculating the coordinate X (respectively Y).

6. Process according to claim 4, characterized in that the same value D is chosen as equal to $X^+ + X^- + Y^+ + Y^-$ for calculating coordinates X and Y.

7. Process according to claim 4, characterized in that the electric signals supplied by the transducers are also combined to produce a digital data item Z, which is a function of the energy of the nuclear radiation received and D is chosen equal to said digital data item Z.

FIG. 1

$X^-$     $A = f(E)$     $A = f(E)$     $X^+$

$-X_0$     $0$     $X_0$     $X$

$X^+_- X^-$     $a_{p-1} \ldots a_0$     $K/D$

$n+p-1$     $p$   $p-1 \ldots 0$     $N-1$     $0$

$O_1$     $O_2$

$\otimes$

$O_1 \times O_2$

BIT DE SIGNE     $n+p+N-1$     $n+p$ $+N-m$     $n+p+N-(m-1)$     $0$

FIG. 2

$X$

$X^+_- X^-$     $K/D$

$n-1$     $0$     $N-1$     $0$

$O_1$     $O_2$

$\otimes$

$O_1 \times O_2$

BIT DE SIGNE     $n+N-1$     $n$ $+N-m$     $n+N-(m-1)$     $0$

$+1?$

$X$

FIG. 3